# EUROPEAN PATENT SPECIFICATION

(11) **EP 2 908 849 B1**
(45) Date of publication and mention of the grant of the patent: **17.04.2019**
(21) Application number: 13832975.0
(22) Date of filing: 30.08.2013
(51) Int. Cl.: A61K 47/64, A61K 38/46, A61K 38/18, A61K 39/395, A61P 35/00, A61P 7/00, A61P 29/00, A61P 25/04

(54) **A METHOD TO IMPROVE PHARMACOKINETICS OF DRUGS**
VERFAHREN ZUR VERBESSERUNG DER PHARMAKOKINETIK VON ARZNEIMITTELN
PROCÉDÉ D'AMÉLIORATION DE LA PHARMACOCINÉTIQUE DE MÉDICAMENTS

(30) Priority: 31.08.2012 US 201261696054 P
(43) Date of publication of application: 26.08.2015
(73) Proprietor: Board of Regents, The University of Texas System, Austin, TX 78701 (US)
(72) Inventor: HARGREAVES, Kenneth M., San Antonio, Texas 78229 (US)
(74) Representative: Arth, Hans-Lothar
(86) International application number: PCT/US2013/057510
(87) International publication number: WO 2014/036393

(56) References cited:
- WO-A1-00/76554
- WO-A1-2010/106082
- WO-A2-95/22992
- WO-A2-2006/078278
- WO-A2-2008/098789
- WO-A2-2010/056919
- WO-A2-2010/056919
- US-A1- 2011 311 545
- US-A1- 2011 311 545
- SHAN JIANG ET AL: "Novel Conjugates of Aspirin with Phenolic Acid as Anti-inflammatory Agents Having Significantly Reduced Gastrointestinal Toxicity", ARCHIV DER PHARMAZIE, vol. 343, 10 March 2010 (2010-03-10), pages 215-221, XP55288372, Weinheim ISSN: 0365-6233, DOI: 10.1002/ardp.200900217
- SHERRI C. YOUNG ET AL: "Investigation of anticholinergic and non-steroidal anti-inflammatory prodrugs which reduce chemically induced skin inflammation", JOURNAL OF APPLIED TOXICOLOGY., vol. 32, no. 2, 11 February 2012 (2012-02-11), pages 135-141, XP055257849, GB ISSN: 0260-437X, DOI: 10.1002/jat.1645
- SHRUTI SAWRAJ ET AL: "Design, synthesis and evaluation of novel indomethacin-flavonoid mutual prodrugs as safer NSAIDs", MEDICINAL CHEMISTRY RESEARCH, BIRKHÄUSER-VERLAG, BOSTON, vol. 20, no. 6, 8 June 2010 (2010-06-08), pages 687-694, XP019914677, ISSN: 1554-8120, DOI: 10.1007/S00044-010-9363-9
- YOUNG ET AL.: 'Investigation of anticholinergic and non-steroidal anti- inflammatory prodrugs which reduce chemically induced skin inflammation' JOURNAL OF APPLIED TOXICOLOGY vol. 32, no. 2, 11 February 2011, pages 135 - 141, XP055257849
- VERMA ET AL.: 'Conjugation of some NSAIDs with 5-phenyl-2-aminothiazole for reduced ulcerogenicity' THAI JOURNAL OF PHARMACEUTICAL SCIENCES vol. 34, no. 2, April 2010, pages 49 - 57, XP055257856
- SAWRAJ ET AL.: 'Design, synthesis and evaluation of novel indomethacin- flavonoid mutual prodrugs as safer NSAIDs' MEDICINAL CHEMISTRY RESEARCH vol. 20, no. 6, 2011, pages 687 - 694, XP019914677
- LIU ET AL.: 'Aptamer-incorporated hydrogels for visual detection, controlled drug release, and targeted cancer therapy' ANALYTICAL AND BIOANALYTICAL CHEMISTRY vol. 402, no. 1, 04 November 2011, pages 187 - 194, XP019992286

## Description

### BACKGROUND OF THE INVENTION

### 1. Field of the Invention

The present invention generally relates to compositions and methods of improving the pharmacokinetic properties of drugs. More specifically, the present invention relates to a compound comprising a pharmacologically active agent, which is an aptamer which acts as an oxidized linoleic acid metabolite inhibitor, a plasma protein binding compound, which is a non-steroidal anti-inflammatory drug (NSAID), and a linker molecule coupling the pharmacologically active agent to the plasma protein binding compound, as defined in the claims. The invention also relates to a composition comprising said compound and the use thereof for treating pain and/or inflammation in a subject.

### 2. Description of the Relevant Art

Inflammation is a protective attempt by the organism to remove the injurious stimuli and to initiate the healing process. Inflammation can be classified as either acute or chronic. Acute inflammation is the initial response of the body to harmful stimuli and is achieved by the increased movement of plasma and leukocytes (especially granulocytes) from the blood into the injured tissues. A cascade of biochemical events propagates and matures the inflammatory response, involving the local vascular system, the immune system, and various cells within the injured tissue. Prolonged inflammation, known as chronic inflammation, leads to a progressive shift in the type of cells present at the site of inflammation and is characterized by simultaneous destruction and healing of the tissue from the inflammatory process.

Acute inflammation is characterized by changes to the vascular system. These changes include vasodilation, increased permeability and the slowing of blood flow. Vasodilation progresses to the capillary level, which brings about a net increase in the amount of blood present. The increased blood causes redness and heat to occur at the site of inflammation. This increased permeability of the vessels results in the movement of plasma into the tissues.

Inflammation can occur during a variety of conditions. Some of the causes of inflammation include burns, chemical irritation, infections, physical injuries (bruising), allergic reactions, radiation (e.g., sunburns), foreign bodies, tumor growth, surgery, and trauma. While inflammation is a common symptom of these conditions, the preferred agent for treatment of each condition is very different. Thus, in order to reduce the symptomatic inflammation, the cause of the inflammation must be addressed. Because inflammation is localized to a specific area of the body, it is desirable to develop methodologies that allow the delivery of the appropriate treatment agent to the area, which may help improve the efficacy of such treatments.
In addition, tissues with increased vascular activity may often require specific delivery of drugs. For example, inflammation associated with tumor growth may offer an opportunity for enhanced delivery of therapeutic agents via increased local vascular activity at the tumor site.

It has already been reported that oligonucleotides are conjugated with a ligand by phosphodiester or phosphorothioate linkages. The resulting ligand-conjugated oligonucleotides bind to protein molecules and possess enhanced pharmacokinetic properties (WO00/76554 A1).

Manoharan et al. (WO 2006 / 078 278 A2) disclosed double-strained oligonucleotide comprising at least one aralkyl ligand which improves the pharmacokinetic properties of the oligonucleotide. It shows exemplary siRNAs duplexes linked to naproxen as aralkyl ligand having *in vitro* luciferase gene silencing activity.

Patwardhan et al. (US 2011 / 0311545 A1) disclosed that compounds which inhibit or minimize the production of oxidized linoleic acid metabolites, may be used for treating pain, shock, inflammatory conditions.

### BRIEF DESCRIPTION OF THE DRAWINGS

Advantages of the present invention will become apparent to those skilled in the art with the benefit of the following detailed description of embodiments and upon reference to the accompanying drawings in which:
FIG. 1 depicts a schematic illustration of an inflammatory process; and
FIG. 2 depicts a schematic illustration of a conjugate. Specific embodiments of the invention are shown by way of example in the drawings and will herein be described in detail. The drawings may not be to scale.

### DETAILED DESCRIPTION OF THE PREFERRED EMBODIMENTS

It is to be understood that the terminology used herein is for the purpose of describing particular embodiments only. The word "may" is used throughout this application in a permissive sense (i.e., having the potential to, being able to), not in a mandatory sense (i.e., must). The term "coupled" means directly or indirectly connected.

### DEFINITIONS

The terms used throughout this specification generally have their ordinary meanings in the art, within the context of the invention, and in the specific context where each term is used. Certain terms are discussed below, or elsewhere in the specification, to provide additional guidance to the practitioner in describing the devices and methods of the invention and how to make and use them. It will be appreciated that the same thing can be said in more than one way. Consequently, alternative language and synonyms may be used for any one or more of the terms discussed herein, nor is any special significance to be placed upon whether or not a term is elaborated or discussed in greater detail herein. Synonyms for certain terms are provided. A recital of one or more synonyms does not exclude the use of other synonyms. The use of examples anywhere in this specification, including examples of any terms discussed herein, is illustrative only.

As used herein the terms "administration," "administering," or the like, when used in the context of providing a pharmaceutical or nutraceutical composition to a subject generally refers to providing to the subject one or more pharmaceutical, "over-the-counter" (OTC) or nutraceutical compositions in combination with an appropriate delivery vehicle by any means such that the administered compound achieves one or more of the intended biological effects for which the compound was administered. By way of example, a composition may be administered by parenteral, subcutaneous, intravenous, intracoronary, rectal, intramuscular, intraperitoneal, transdermal, or buccal routes of delivery. Alternatively, or concurrently, administration may be by the oral route. The dosage administered will be dependent upon the age, health, weight, and/or disease state of the recipient, kind of concurrent treatment, if any, frequency of treatment, and/or the nature of the effect desired. The dosage of pharmacologically active compound that is administered will be dependent upon multiple factors, such as the age, health, weight, and/or disease state of the recipient, concurrent treatments, if any, the frequency of treatment, and/or the nature and magnitude of the biological effect that is desired.

As used herein, the term "agonist" generally refers to a type of ligand or drug that binds and alters the activity of a receptor.

As used herein, the term "antagonist" generally refers to a type of receptor ligand which binds a receptor but which does not alter the activity of the receptor; however when used with an agonist, prevents the binding of the agonist to the receptor hence the effect of the agonist.

As used herein, the term "allodynia" generally refers to pain from stimuli which are not normally painful. The pain may occur other than in the area stimulated. Allodynia may generally refer to a heightened pain state.

As used herein, the term "antinociception" generally refers to a reduction in pain sensitivity.

As used herein, the term "monoclonal antibody" generally refers to an antibody obtained from a population of substantially homogeneous antibodies (the individual antibodies comprising the population are identical except for possible naturally occurring mutations that may be present in minor amounts). As used herein, the term "polyclonal antibody" generally refers to a population of antibodies that are directed against a common epitope but which are not identical in structure.

As used herein, terms such as "pharmaceutical composition," "pharmaceutical formulation," "pharmaceutical preparation," or the like, generally refer to formulations that are adapted to deliver a prescribed dosage of one or more pharmacologically active compounds to a cell, a group of cells, an organ or tissue, an animal or a human. Methods of incorporating pharmacologically active compounds into pharmaceutical preparations are widely known in the art. The determination of an appropriate prescribed dosage of a pharmacologically active compound to include in a pharmaceutical composition in order to achieve a desired biological outcome is within the skill level of an ordinary practitioner of the art. A pharmaceutical composition may be provided as sustained-release or timed-release formulations. Such formulations may release a bolus of a compound from the formulation at a desired time, or may ensure a relatively constant amount of the compound present in the dosage is released over a given period of time. Terms such as "sustained release" or "timed release" and the like are widely used in the pharmaceutical arts and are readily understood by a practitioner of ordinary skill in the art. Pharmaceutical preparations may be prepared as solids, semi-solids, gels, hydrogels, liquids, solutions, suspensions, emulsions, aerosols, powders, or combinations thereof. Included in a pharmaceutical preparation may be one or more carriers, preservatives, flavorings, excipients, coatings, stabilizers, binders, solvents and/or auxiliaries that are, typically, pharmacologically inert. It will be readily appreciated by an ordinary practitioner of the art that, pharmaceutical compositions, formulations and preparations may include pharmaceutically acceptable salts of compounds. It will further be appreciated by an ordinary practitioner of the art that the term also encompasses those pharmaceutical compositions that contain an admixture of two or more pharmacologically active compounds, such compounds being administered, for example, as a combination therapy.

As used herein the term "pharmaceutically acceptable salts" includes salts prepared from by reacting pharmaceutically acceptable non-toxic bases or acids, including inorganic or organic bases, with inorganic or organic acids. Pharmaceutically acceptable salts may include salts derived from inorganic bases include aluminum, ammonium, calcium, copper, ferric, ferrous, lithium, magnesium, manganic salts, manganous, potassium, sodium, zinc, etc. Examples include the ammonium, calcium, magnesium, potassium, and sodium salts. Salts derived from pharmaceutically acceptable organic non-toxic bases include salts of primary, secondary, and tertiary amines, substituted amines including naturally occurring substituted amines, cyclic amines, and basic ion exchange resins, such as arginine, betaine, caffeine, choline, N,N'-dibenzylethylenediamine, diethylamine, 2-dibenzylethylenediamine, 2-diethylaminoethanol, 2-dimethylaminoethanol, ethanolamine, ethylenediamine, N-ethyl-morpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine, etc.

The terms "reducing," "inhibiting" and "ameliorating," as used herein, when used in the context of modulating a pathological or disease state, generally refers to the prevention and/or reduction of at least a portion of the negative consequences of the disease state. When used in the context of an adverse side effect associated with the administration of a drug to a subject, the term(s) generally refer to a net reduction in the severity or seriousness of said adverse side effects.

As used herein the term "subject" generally refers to a mammal, and in particular to a human.

As used herein, the term "treat" generally refers to an action taken by a caregiver that involves substantially inhibiting, slowing or reversing the progression of a disease, disorder or condition, substantially ameliorating clinical symptoms of a disease disorder or condition, or substantially preventing the appearance of clinical symptoms of a disease, disorder or condition.

Terms such as "in need of treatment," "in need thereof," "benefit from such treatment," and the like, when used in the context of a subject being administered a pharmacologically active composition, generally refers to a judgment made by an appropriate healthcare provider that an individual or animal requires or will benefit from a specified treatment or medical intervention. Such judgments may be made based on a variety of factors that are in the realm of expertise of healthcare providers, but include knowledge that the individual or animal is ill, will be ill, or is at risk of becoming ill, as the result of a condition that may be ameliorated or treated with the specified medical intervention.

By "therapeutically effective amount" is meant an amount of a drug or pharmaceutical composition that will elicit at least one desired biological or physiological response of a cell, a tissue, a system, animal or human that is being sought by a researcher, veterinarian, physician or other caregiver.

The term "OLAM inhibitor" is used to describe a compound that inhibits and/or minimizes the production of oxidized linoleic acid metabolites and/or blocks the activity of oxidized linoleic acid metabolites.

### Plasma Protein Coupled Pharmacologically Active Agents

Many drugs have limitations in their pharmacokinetic properties due to rapid renal filtration, degradation by circulating enzymes or poor penetration into inflamed tissue. Among this list of drugs with suboptimal pharmacokinetic properties are aptamers, tioconazole, nifedipine, metoprolol, and others. Many of the major physiological responses of acute inflammation are vascular in nature and include plasma extravasation and vasodilation. Plasma extravasation is the outflow of fluid and plasma proteins into the inflamed extracellular compartment. Therefore, agents that are bound to plasma proteins are likely to exhibit reduced renal filtration, reduced exposure to circulating enzymes and/or increased delivery into inflamed tissues by the process of plasma extravasation.

Many anti-inflammatory drugs are heavily bound to plasma proteins. Indeed, most nonsteroidal anti-inflammatory drugs (NSAIDs), including flurbiprofen are >99% bound to plasma proteins. Thus, only ∼0.1% of the total flurbiprofen concentration in plasma is in the "free" (unbound) state. Because only the free concentration is biologically active, physiological responses that alter delivery of plasma proteins or binding of these drugs to plasma proteins are likely to alter the magnitude of the pharmacological effect.

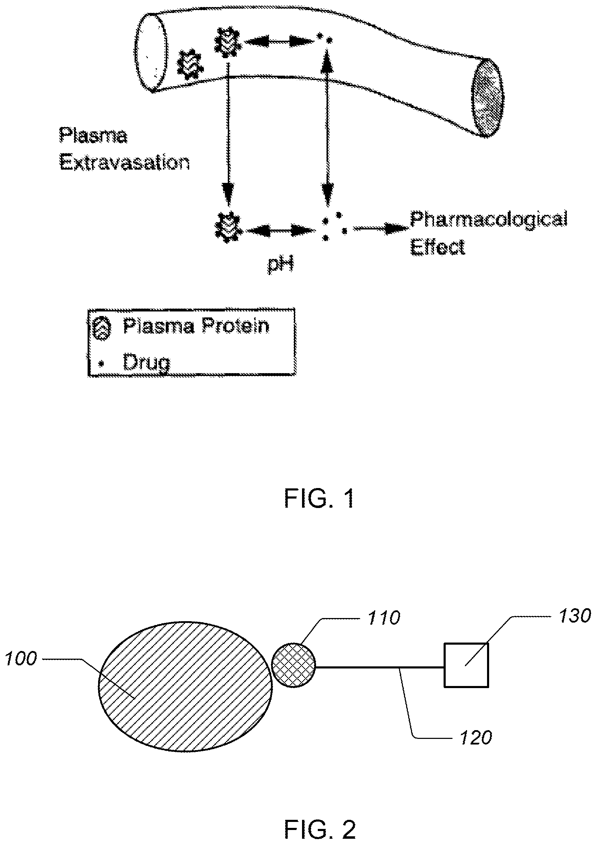

Our studies indicate that: inflammation alters the delivery of drugs to the inflamed tissue; activation of capsaicin-sensitive nerves increases the content of protein-bound drugs; and that reduced pH increases free drug concentrations of the protein-bound drugs. Thus, alterations in both plasma extravasation and tissue pH seem to be relevant factors regulating the delivery and bioavailability of anti-inflammatory drugs and other compounds which are highly protein-bound. FIG. 1 depicts a schematic illustration of an inflammatory process. Drugs that are heavily bound to plasma proteins are released into extracellular space primarily by the process of plasma extravasation. Both vasodilation (e.g., blood flow) and vascular permeability regulate plasma extravasation. The drug is distributed between free (i.e. "unbound") and bound (i.e. plasma protein:drug complex) states. Only the free drug concentration is pharmacologically active. Factors that influence drug binding to plasma proteins will alter free drug concentration and therefore would be predicted to alter the magnitude of the pharmacological effect. Thus, both plasma extravasation and local tissue ion concentrations such as altered pH in inflamed areas would be expected to alter the efficacy of protein-bound drugs.

Inflammation of tissue and/or activation of capsaicin-sensitive nerves within tissues (tissue pain states) can occur due to a number of different causes, all requiring a specific and different treatment. Many of the treatments involve drugs that may be poorly solubilized, and have limited availability in the sites of inflammation and tissue pain states. A method has been developed that 1) improves pharmacokinetic properties of drugs and 2) enhances delivery of drugs to inflamed, painful or injured tissue. The basic methodology is to conjugate agents with high plasma protein binding properties (a "plasma protein binding compound") to one or more other selected drug(s) of interest. This will effectively confer increased plasma binding properties to the drug or drugs of interest and result in targeting of agents to body sites where above normal states of vasodilation and vascular permeability (and thus above normal levels of plasma extravasation) exist. A schematic diagram of the conjugate is depicted in FIG. 2. A therapeutic agent of interest **130** may be coupled to a plasma protein **100** through a plasma protein binding compound **110** and, optionally, through a linker **120** which couples the therapeutic agent **130** to the plasma protein binding compound **110.**

Examples of diseases or medical conditions involving stressed, inflamed, painful and/or traumatized tissues and cells include ischemic tissue conditions including ischemic heart disease, myocardial infarction, cancer, burns, traumatic tissue injury, arthritis, surgery-induced tissue damage, infections, cerebrovascular accidents, and other conditions involving a disruption in cellular membrane integrity.

For the treatment of inflammation a number of pharmacologically active agents may be used, depending on the cause of the inflammation. Examples of drug classes used to treat inflammation include:
antibiotics; growth factors; local anesthetics;
analgesics; and antihistamines. Any pharmacologically active agent from these drug classes may be coupled to a plasma protein binding compound to enhance the delivery of these drugs to the inflamed, painful and/or injured body site. With respect to pain and inflammation management, additional benefits may be gained if the plasma protein binding compound is also an anti-inflammatory compound, or has pain reducing properties. Examples of compounds that are plasma protein binding compounds and are pharmacologically active in inflamed, injured and/or painful tissue include non-steroidal anti-inflammatory drugs ("NSAIDS"), antibiotics, local anesthetics, opiates, opioids, or steroids. Specific examples of NSAIDS that are plasma binding compounds include, but are not limited to: salicylates (e.g., Aspirin (acetylsalicylic acid), diflunisal, and salsalate); propionic acid derivatives (e.g., ibuprofen, dexibuprofen, naproxen, fenoprofen, ketoprofen, dexketoprofen, flurbiprofen, oxaprozin, and loxoprofen); acetic acid derivatives (e.g., indomethacin, tolmetin, sulindac, etodolac, ketorolac, diclofenac, and nabumetone); enolic acid (oxicam) derivatives (e.g., piroxicam, meloxicam, tenoxicam, droxicam, lornoxicam, and isoxicam); and fenamic acid derivatives (fenamates) (e.g., mefenamic acid; meclofenamic acid; flufenamic acid; and tolfenamic acid. Other exemplary plasma protein binding compounds that also exhibit pain reducing properties or anti-inflammatory properties are antibiotics (e.g., clindamycin, erythromycin, or the sulphonamides), local anesthetics (e.g., bupivacaine), opiates (e.g., methadone), or steroids (e.g., prednisolone).

For the treatment of other conditions such as cancer, myocardial infarction, cerebrovascular accidents, ulcers, etc, the increased vascular permeability provides the opportunity for local delivery of high concentrations of drugs that are bound to plasma proteins. Examples of drug classes used to treat these conditions include inhibitors of tissue plasminogen activator, anti-cancer drugs including chemothcrapeutics or drugs that alter angiogenesis, antiulcer drugs, and so on.

In an embodiment, the plasma protein binding compound, will bind to the plasma proteins in a pH-dependent fashion, such that binding would be reduced at the lower pH values (e.g., at pH of less than 7) seen in tissue inflammation, cancer, injury and tissue hypoxia. This would lead to increased free drug concentrations in the inflamed tissue and therefore improved pharmacodynamics. Examples of drugs that bind to plasma proteins in a pH-dependent fashion are biperiden, clindamycin, dexamethasone, fluoxetine, and nefinavir.

With regard to hypoxia, it is known that hypoxia leads to inflammation in some diseases/disorders involving tissues. Hypoxia in tissues leads to lower pH. As noted above, in some embodiments, the protein binding compound may have reduced binding to the plasma protein at reduced levels. Thus, the use of pH dependent complexes of protein binding compounds and therapeutic agents allows an effective therapy for treating systemic shock and other conditions associated with hypoxic conditions (re-perfusion injuries, decreasing myocardial damage post-myocardial infarction and other conditions).

In another example, plasma bound antioxidants may be used for the treatment of tissue reperfusion injuries caused by free radicals in hypoxic tissues which are suddenly re-oxygenated. For example, antioxidants such as NDGA, Vitamin C, glutathione, resveratrol, vitamin E, β-carotene, and astaxanthin may be bound to a plasma protein either directly or through a plasma protein binding compound to diseases associated with reactive oxygen radical production. Clinical situations where this occurs include : 1.) post acute myocardial infarction to save as much stunned and ischemic myocardium as possible; 2.) reperfusion injury post-organ transplant; and 3.) severed limbs which are microsurgically attached.

The pharmacologically active agent used to treat the inflammatory condition may be coupled to the plasma protein binding compounds using a number of techniques. In an embodiment, a pharmacologically active agent may be directly covalently linked to a plasma protein binding compound. For example, ribonucleotide or nucleotide-based drugs (e.g., aptamers) typically include one or more carboxylic acid functional groups and one or more amino functional groups. Any of the reactive carboxylic acid groups or reactive amino groups can be used to form a covalent bond to functional groups on the plasma protein binding compound. For example, many NSAIDS, and other compounds that bind to plasma proteins, have a free carboxylic acid group which can be covalently linked to an aptamer through an amide linkage. A free amino group of the aptamer may be linked to the carboxylic acid group using standard reactions for forming amino acid linkages.

Small molecule drugs may also be covalently linked to a plasma protein binding compound. Reactive functional groups on the small molecule drugs may be coupled with reactive functional groups on a plasma protein binding compound. For example, many NSAIDS, and other compounds that bind to plasma proteins, have a free carboxylic acid group which can be covalently linked to reactive alcohol groups, amino groups, or thiol groups on the small molecule drugs.

In some embodiments, it may be desired to use a linker molecule to couple the pharmacologically active agent used to treat the inflammatory condition to a plasma protein binding compound. A linker molecule is generally any molecule that is used to covalently couple the drug to the plasma protein binding compound. In some examples, a linker may be a homobifunctional linker. Such compounds may have the general formula R-(CH₂)ₙ-R, where R is CO₂H, NH₂, OH, SH, CH=O, CR¹=O, CH=NH, or halogen; n is 1-10, and R¹ is C₁-C₆ alkyl). Alternatively, the linker may be a heterobifunctional linker. Such compounds may have the general formula R²(CH₂)ₙ, - R³, where R² and R³ are different, and where each R² and R³ is CO₂H, NH₂, OH, SH, CH=O, CR¹=O, CH=NH, or halogen; n is 1-10, and R¹ is C₁-C₆ alkyl. A linker molecule may covalently bond with at least one reactive functional group of the drug and at least one reactive functional group of the plasma protein binding compound. Specific linkers may be chosen for use in the plasma protein - plasma protein binding compound - linker - drug complex such that drug release may be optimized for specific ionic conditions, such as a certain pH or pH range.

In another example, a pharmacologically active agent used to treat the inflammatory condition may be bound to a plasma protein binding compound by a linker molecule that is removed by enzymes present in inflamed tissue. In such examples, the linked plasma protein binding compound helps to transport the drug to the inflamed tissue. Once the complex reaches the inflamed tissue, the linker is removed by the enzymes present at the site of inflammation.

In other examples, a plasma protein binding compound may be bound to one or more other compounds which may or may not themselves be bound, the resulting structure yielding multi-valent target binding capability. For example, the plasma protein binding compound may be bound to two or more therapeutic compounds, each of the therapeutic compounds being specific for a different target. In this way a single complex may be designed to address multiple biological pathways that contribute to the disease state.

In an example, the plasma protein binding compound may not be a pharmacologically active compound. In such situations, the plasma protein binding compound is simply used to transport the drug into the plasma and to the site of inflammation. According to the invention, the plasma protein binding compound is a non-steroidal anti-inflammatory drug (NSAID), preferably ibuprofen.

### Enhancement of OLAM Inhibitors

TRPV1, also known as the capsaicin receptor, plays a pivotal role in bum injury and other important pain conditions by evoked hyperalgesia and allodynia such that the mice deficient in TRPV1 protein show little to no heat hyperalgesia in these models. The key role played by TRPV1 in the development of thermal hyperalgesia and possibly mechanical hyperalgesia in various pain models is well established in animal and human studies. Signaling cascades initiated by a variety of inflammatory mediators may sensitize TRPV1 and contribute to inflammatory hyperalgesia. Given the importance of TRPV1 in inflammatory pain, burn pain and cancer pain, including other various pain states, a number of groups in the past have developed antagonists against TRPV1 as potential treatments for pain and/or inflammatory conditions. Unfortunately, it was discovered that antagonism of the TRPV1 receptor with TRPV1 antagonists can lead to sometimes dangerous levels of hyperthermia. This insight has led to the search for endogenous targets upstream of the TRPV1 receptor which, if eliminated, immunoneutralized or otherwise interfered with, might alleviate pain and; or inflammatory conditions.

Ours was the first group to demonstrate that oxidized metabolites of linoleic acid act as ligands at the TRP-class of neurons, and in the case of the TRPV1 receptor, Oxidized Linoleic Acid Metabolites (OLAMs) are TRPV1 agonists and mediate pain and/or inflammatory conditions. Linoleic acid is also known by its TUPAC name *cis, cis*-9,12-octadecadienoic acid. Linoleic acid has a structure:

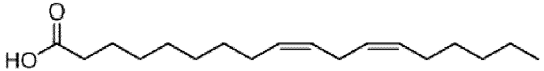

Pharmacological interventions that can block the generation of the endogenous TRPV1 ligand in response to heat may be of therapeutic use. Oxidized linoleic acid metabolites are generated upon heat stimulation of skin. Oxidized linoleic acid metabolites include oxo linoleic acid metabolites, hydroxyl linoleic acid metabolites, and epoxy linoleic acid metabolites. Examples of oxo linoleic acid metabolites include (10E,12Z)-9-oxooctadeca-10,12-dienoic acid (9-oxoODE, 9-KODE) and (9Z,11E)-13-oxooctadeca-9,11-dienoic acid (13-oxoODE, 13-KODE). Examples of hydroxyl linoleic acid metabolites include 9-hydroxyoctadecadienoic acid (9-HODE); 13-hydroxyoctadecadienoic acid (13-HODE); 9(10)-dihydroxy-octadec-12-enoic acid (9,10-DiHOME); and 12,13-dihydroxy-9Z-octadecenoic acid (12,13-DiHOME). Examples of epoxy linoleic acid metabolites include (12Z)-9,10-epoxyoctadecenoic acid (9(10)-EpOME) and 12,13-epoxyoctadec-9Z-enoic acid (12(13)-EpOME). It is believed that oxidized linoleic acid metabolites may function as endogenous TRPV1 agonists.

The blockade of synthesis or immunoneutralization of oxidized linoleic acid metabolites results in decreased activation of pain sensing neurons by heat *in vitro* and results in thermal antinociception *in vivo.* Immunoneutralization of oxidized linoleic acid metabolites may be accomplished by the use of one or more antibodies that bind to at least one oxidized linoleic acid metabolite. Antibodies for oxidized linoleic acids may be formed using the procedure of Spindler et al. (Spindler et al., "Significance and immunoassay of 9- and 13-hydroxyoctadecadienoic acids." Biochein Biophys Res Commun. 1996; 218:187-191). Antibodies for oxidized linoleic acids may be monoclonal antibodies or polyclonal antibodies.

The blockade of synthesis or immunoneutralization of oxidized linoleic acid metabolites results in decreased activation of pain sensing neurons by heat *in vitro* and results in thermal antinociception *in vivo.* Immunoneutralization of oxidized linoleic acid metabolites may be accomplished by the use of one or more aptamers that bind to at least one oxidized linoleic acid metabolite.

Recent research has indicated that activation of TRPVI by 9-HODE may have other roles in the body depending upon the expression of TRPV1. TRPV1 in the spinal cord may play an important role in maintenance of thermal and mechanical allodynia in inflammatory or other pain conditions. Depolarization of the spinal cord may lead to the release of 9-HODE and activation of TRPV1. 9-HODE in the spinal cord may lead to development of mechanical allodynia. Similar to heated skin, depolarized spinal cord (with high potassium) may release compound(s) that have TRPV1 agonist activity. Depolarized spinal cord superfusate may contain significantly higher amounts of 9-HODE. Moreover, activation of TRPV1 in the spinal cord by capsaicin (positive control) or by 9-HODE results in tactile allodynia that is completely reversible by a TRPVI antagonist. Thus, in some embodiments, the role of 9-HODE and similar linoleic acid oxidation products extends beyond heat-nociception.

In some embodiments, a method may include treating inflammation.

To improve the pharmacokinetic properties of OLAM inhibitors, an OLAM inhibitor may be coupled to an agent with plasma protein binding properties ("plasma protein binding compounds"). This will confer increased plasma binding properties to the OLAM inhibitor, allowing the OLAM inhibitor to be carried to the site of inflammation through plasma extravasation. Once the plasma protein bound OLAM inhibitor arrives at the site of inflammation, the inhibitor may become unbound from the plasma protein due to the low pH generally associated with tissue inflammation or injury.

In an example ibuprofen, a drug that is more than 99% bound to plasma proteins, may be linked to an aptamer (that acts as an OLAM inhibitor), a class of drugs that demonstrates poor plasma protein binding properties. This combination would decrease renal filtration of the aptamer (since plasma proteins are not filtered), decrease degradation by circulating enzymes, and increase delivery to inflamed tissue due to plasma extravasation of plasma proteins into areas of tissue injury. A compound according to the present invention comprises a pharmacologically active agent, which is an aptamer which acts as an oxidized linoleic acid metabolite inhibitor, wherein the oxidized linoleic acid metabolite is a hydroxyl linoleic acid metabolite selected from 9-hydroxyoctadecadienoic acid (9-HODE), 13-hydroxyoctadecadienoic acid (13-HODE), 9(10)-dihydroxy-octadec-12-enoic acid (9,10-DiHOME) or 12, 13-dihydroxy-9Z-octadecenoic acid (12,13-DiHOME), a plasma protein binding compound, which is a non-steroidal anti-inflammatory drug (NSAID), and a linker molecule coupling the pharmacologically active agent to the plasma protein binding compound. The invention also relates to a pharmaceutical composition comprising said compound and the use thereof for treatment of pain and/or inflammatory conditions.

### Pharmaceutical Compositions

Any suitable route of administration may be employed for providing a subject with an effective dosage of the compounds described herein. For example, oral, rectal, topical, parenteral, ocular, pulmonary, nasal, and the like may be employed. Dosage forms include tablets, troches, dispersions, suspensions, solutions, capsules, creams, ointments, aerosols, and the like.

The compounds described herein may be present in pharmaceutical compositions suitable for oral, rectal, topical, parenteral (including subcutaneous, intramuscular, and intravenous), ocular (ophthalmic), pulmonary (aerosol inhalation), or nasal administration, although the most suitable route in any given case will depend on the nature and severity of the conditions being treated and on the nature of the active ingredient. They may be conveniently presented in unit dosage form and prepared by any of the methods well-known in the art of pharmacy.

In practical use, compositions may be combined as the active ingredient in intimate admixture with a pharmaceutical carrier according to conventional pharmaceutical compounding techniques. The carrier may take a wide variety of forms depending on the form of preparation desired for administration, e.g., oral or parenteral (including intravenous). In preparing the compositions for oral dosage form, any of the usual pharmaceutical media may be employed, such as, for example, water, glycols, oils, alcohols, flavoring agents, preservatives, coloring agents and the like in the case of oral liquid preparations, such as, for example, suspensions, elixirs and solutions; or carriers such as starches, sugars, microcrystalline cellulose, diluents, granulating agents, lubricants, binders, disintegrating agents and the like in the case of oral solid preparations such as, for example, powders, capsules and tablets, with the solid oral preparations being preferred over the liquid preparations. Because of their ease of administration, tablets and capsules represent the most advantageous oral dosage unit form in which case solid pharmaceutical carriers are obviously employed. If desired, tablets may be coated by standard aqueous or nonaqueous techniques.

The pharmaceutical preparations may be manufactured in a manner which is itself known to one skilled in the art, for example, by means of conventional mixing, granulating, dragee-making, softgel encapsulation, dissolving, extracting, or lyophilizing processes. Thus, pharmaceutical preparations for oral use may be obtained by combining the compositions with solid and semi-solid excipients and suitable preservatives, and/or co-antioxidants. Optionally, the resulting mixture may be ground and processed. The resulting mixture of granules may be used, after adding suitable auxiliaries, if desired or necessary, to obtain tablets, softgels, lozenges, capsules, or dragee cores.

Suitable excipients may be fillers such as saccharides (e.g., lactose, sucrose, or mannose), sugar alcohols (e.g., mannitol or sorbitol), cellulose preparations and/or calcium phosphates (e.g., tricalcium phosphate or calcium hydrogen phosphate). In addition binders may be used such as starch paste (e.g., maize or corn starch, wheat starch, rice starch, potato starch, gelatin, tragacanth, methyl cellulose, hydroxypropylmethylcellulose, sodium carboxymethylcellulose, and/or polyvinyl pyrrolidone). Disintegrating agents may be added (e.g., the above-mentioned starches) as well as carboxymethyl-starch, cross-linked polyvinyl pyrrolidone, agar, or alginic acid or a salt thereof (e.g., sodium alginate). Auxiliaries are, above all, flow-regulating agents and lubricants (e.g., silica, talc, stearic acid or salts thereof, such as magnesium stearate or calcium stearate, and/or polyethylene glycol, or PEG). Dragee cores are provided with suitable coatings, which, if desired, are resistant to gastric juices. Soft gelatin capsules ("softgels") are provided with suitable coatings, which, typically, contain gelatin and/or suitable edible dye(s). Animal component-free and kosher gelatin capsules may be particularly suitable for the embodiments described herein for wide availability of usage and consumption. For this purpose, concentrated saccharide solutions may be used, which may optionally contain gum arabic, talc, polyvinyl pyrrolidone, polyethylene glycol (PEG) and/or titanium dioxide, lacquer solutions and suitable organic solvents or solvent mixtures, including dimethylsulfoxide (DMSO), tetrahydrofuran (THE), acetone, ethanol, or other suitable solvents and co-solvents. In order to produce coatings resistant to gastric juices, solutions of suitable cellulose preparations such as acetylcellulose phthalate or hydroxypropylmethyl-cellulose phthalate, may be used. Dye stuffs or pigments may be added to the tablets or dragee coatings or soft gelatin capsules, for example, for identification or in order to characterize combinations of active compound doses, or to disguise the capsule contents for usage in clinical or other studies.

In some examples, the compounds will typically be formulated in such vehicles at a concentration of about 0.1 mg/ml to 100 mg/ml.

For the prevention or treatment of pain or inflammation, the appropriate dosage of the composition will depend on the type of the severity and location of the pain or inflammation, whether the compositions are administered for preventive or therapeutic purposes, previous therapy, the patient's clinical history and response to the composition, and the discretion of the attending physician. The composition is suitably administered to the patient at one time or over a series of treatments.

## Claims

1. A compound comprising:
a pharmacologically active agent, wherein the pharmacologically active agent is an aptamer which acts as an oxidized linoleic acid metabolite inhibitor, the oxidized linoleic acid metabolite is a hydroxyl linoleic acid metabolite,
a plasma protein binding compound, and
a linker molecule coupling the pharmacologically active agent to the plasma protein binding compound,
wherein the plasma protein binding compound is a non-steroidal anti-inflammatory drug (NSAID); wherein the hydroxyl linoleic acid metabolite is 9-hydroxyoctadecadienoic acid (9-HODE), 13-hydroxyoctadecadienoic acid (13-HODE), 9(10)-dihydroxy-octadec-12-enoic acid (9,10-DiHOME) or 12, 13-dihydroxy-9Z-octadecenoic acid (12,13-DiHOME).

2. The compound of claim 1, wherein the non-steroidal anti-inflammatory drug (NSAID) is ibuprofen.

3. The compound of any of claims 1 - 2, wherein the hydroxyl linoleic acid metabolite is 9-hydroxyoctadecadienoic acid (9-HODE).

4. The compound of claim 1, wherein the pharmacologically active agent is used to treat an inflammatory condition.

5. The compound of claim 1, wherein the pharmacologically active agent is used to treat a pain condition.

6. The compound of claim 2, wherein the ibuprofen molecule binds to plasma proteins in a pH-dependent fashion.

7. The compound of claim 2, wherein binding of the ibuprofen molecule to plasma proteins is reduced at pH lower than 7.

8. A pharmaceutical composition comprising one or more compounds as described in any one of claims 1 - 7.

9. The pharmaceutical composition according to claim 8 for use in the treatment of pain, inflammatory conditions, or combinations thereof.

## Patentansprüche

1. Eine Verbindung umfassend:
einen pharmakologisch aktiven Wirkstoff, wobei der pharmakologisch aktive Wirkstoff ein Aptamer ist, der als Inhibitor des oxidierten Linolsäure-Metaboliten wirkt, der oxidierte Linolsäure-Metabolit ein Hydroxy-Linolsäure-Metabolit ist,
eine plasmaproteinbindende Verbindung, und
ein Linker-Molekül, das den pharmakologisch aktiven Wirkstoff mit der plasmaproteinbindenden Verbindung verknüpft,
wobei die plasmaproteinbindende Verbindung ein nicht-steroidales antiinflammatorisches Arzneimittel (NSAID) ist;
wobei der Hydroxy-Linolsäure-Metabolit 9-Hydroxyoctadecadiensäure (9-HODE), 13-Hydroxyoctadecadiensäure (13-HODE), 9(10)-Dihydroxy-octadec-12-ensäure (9,10-DiHOME) oder 12,13-Dihydroxy-9Z-octadecansäure (12,13-DiHOME) ist.

2. Die Verbindung nach Anspruch 1, wobei das nicht-steroidale anti-inflammatorische Arzneimittel (NSAID) Ibuprofen ist.

3. Die Verbindung nach einem der Ansprüche 1 - 2, wobei der Hydroxy-Linolsäure-Metabolit 9-Hydroxyoctadecadiensäure (9-HODE) ist.

4. Die Verbindung nach Anspruch 1, wobei der pharmakologisch aktive Wirkstoff verwendet wird, um einen Entzündungszustand zu behandeln.

5. Die Verbindung nach Anspruch 1, wobei der pharmakologisch aktive Wirkstoff verwendet wird, um einen Schmerzzustand zu behandeln.

6. Die Verbindung nach Anspruch 2, wobei das Ibuprofen-Molekül in einer pHabhängigen Weise an Plasmaproteine bindet.

7. Die Verbindung nach Anspruch 2, wobei die Bindung des Ibuprofen-Moleküls an Plasmaproteine bei einem pH-Wert von weniger als 7 reduziert wird.

8. Eine pharmazeutische Zusammensetzung umfassend eine oder mehr Verbindungen wie beschrieben in einem der Ansprüche 1 - 7.

9. Die pharmazeutische Zusammensetzung nach Anspruch 8 zur Verwendung bei der Behandlung von Schmerzen, Entzündungszuständen oder Kombinationen davon.

## Revendications

1. Un composé comprenant :
un principe actif pharmacologique étant un aptamère agissant comme inhibiteur de métabolite d'acide linoléique oxydé, ledit métabolite d'acide linoléique oxydé étant un métabolite hydroxydé d'acide linoléique,
un composé se liant aux protéines plasmatiques, et
une molécule de liaison liant le principe actif pharmacologique au composé se liant aux protéines plasmatiques,
où le composé se liant aux protéines plasmatiques est un médicament anti-inflammatoire non stéroïdien (AINS) ;
où le métabolite hydroxydé d'acide linoléique est un acide 9-hydroxyoctadécadiénoïque (9-HODE), un acide 13-hydroxyoctadécadiénoïque (13-HODE), un acide 9(10)-dihydroxy-octadéca-12-énoïque (9,10-DiHOME) ou un acide 12,13-dihydroxy-9Z-octadécénoïque (12,13-DiHOME).

2. Le composé de la revendication 1, où le médicament anti-inflammatoire non stéroïdien (AINS) est l'ibuprofène.

3. Le composé de l'une des revendications 1 ou 2, où le métabolite hydroxydé d'acide linoléique est un acide 9-hydroxyoctadécadiénoïque (9-HODE).

4. Le composé de la revendication 1, où le principe actif pharmacologique est utilisé pour traiter une inflammation.

5. Le composé de la revendication 1, où le principe actif pharmacologique est utilisé pour traiter une douleur.

6. Le composé de la revendication 2, où la molécule d'ibuprofène se lie aux protéines plasmatiques en fonction du pH.

7. Le composé de la revendication 2, où la liaison de la molécule d'ibuprofène aux protéines plasmatiques est réduite lorsque le pH est inférieur à 7.

8. Une formule pharmaceutique comprenant au moins un composé décrit dans l'une des revendications 1 à 7.

9. La formule pharmaceutique conforme à la revendication 8 , destinée au traitement de la douleur, des inflammations ou d'une combinaison des deux.
